# EUROPÄISCHE PATENTSCHRIFT

(11) **EP 1 478 680 B1**
(45) Veröffentlichungstag und Bekanntmachung des Hinweises auf die Patenterteilung: **15.08.2007**
(21) Anmeldenummer: 03704623.2
(22) Anmeldetag: 14.02.2003
(51) Int. Cl.: C08G 69/10, C07D 301/02

(54) **POLYAMINOSÄUREN UND VERFAHREN ZU DEREN HERSTELLUNG**
POLYAMINO ACIDS AND METHOD FOR PRODUCING THE SAME
POLYAMINOACIDES ET LEUR PROCEDE DE FABRICATION

(30) Priorität: 19.02.2002 DE 10206793
(43) Veröffentlichungstag der Anmeldung: 24.11.2004
(73) Patentinhaber: Saltigo GmbH, 51369 Leverkusen (DE)
(72) Erfinder: GELLER, Thomas, 51519 Odenthal (DE); GERLACH, Arne, 51519 Odenthal (DE); VIDAL-FERRAN, Anton, 43340 Montbrio del Camp (ES); MILITZER, Hans-Christian, 51519 Odenthal (DE); LANGER, Reinhard, 47918 Tönisvorst (DE)
(74) Vertreter: Wichmann, Birgid
(86) Internationale Anmeldenummer: PCT/EP2003/001512
(87) Internationale Veröffentlichungsnummer: WO 2003/070808

(56) Entgegenhaltungen:
- EP-A- 1 006 127
- WO-A-96/33183
- US-A- 2 789 973
- US-A- 5 780 579
- EBRAHIM S ET AL: "Synthetic applications of polymeric alpha-amino acids" TETRAHEDRON: ASYMMETRY, ELSEVIER SCIENCE PUBLISHERS, AMSTERDAM, NL, Bd. 8, Nr. 19, 1. Oktober 1997 (1997-10-01), Seiten 3163-3173, XP004092596 ISSN: 0957-4166

## Beschreibung

Die Erfindung betrifft Polyaminosäuren, ein Verfahren zu ihrer Herstellung sowie ihre Verwendung als Katalysatoren für die enantioselektive Epoxidierung.

Chiral, nicht-racemische Epoxide sind wertvolle Bausteine für die Herstellung von optisch aktiven Wirkstoffen und Materialien (z.B. a) Bioorg. Med. Chem., 1999, 7, 2145-2156; b) Tetrahedron Lett., 1999, 40, 5421-5424). Die größte Aufmerksamkeit wurde in der Literatur der Epoxidierungsmethode von Juliä und Colonna gewidmet, die zeigen konnten, dass enantiomeren- und diastereomerenangereicherte Polyaminosäuren (PAS) in Gegenwart von wässriger Wasserstoffperoxid- und NaOH-Lösung sowie eines Aromaten bzw. halogenierten Kohlenwasserstoffs als Lösungsmittel in der Lage sind, die enantioselektive Epoxidierung von α,β-ungesättigten Enonen zu katalysieren (Angew., Chem., Int. Ed. Eng., 1980,19, 929-930).

Für die Herstellung von Polyaminosäuren wurden verschiedene Verfahren beschrieben (z.B. Adcances in Protein Chemistry, 1958, 13, 243-492; Russ. Chem. Rev., 1965, 34, 329; Comprehensive Chemical Kinetics, 1976, 15, 583-637). Die meisten Verfahren nutzen das Prinzip der statistischen Polymerisation, bei der die N-Carboxyanhydride (NCA) der entsprechenden Aminosäuren mit einem Initiator (z.B. Amine, Wasser, Alkohole und Alkoholate) in einem inerten Lösungsmittel (z.B. Acetonitril, Dioxan, THF, Benzol) umgesetzt werden. Hierbei wird eine Mischung aus Polyaminosäuren mit verschiedenen Kettenlängen erhalten, als Hautprodukt wird eine Polyaminosäure mit der Kettenlänge angenommen, die sich aus dem molekularen Verhältnis zwischen NCA und Initiator errechnet. Die Polymerisationen werden meist bei Raumtemperatur ausgeführt. Beispiele für die Polymerisationen von NCAs bei erhöhter Temperatur sind hingegen selten. So wurden beispielsweise hochmolekulare Filme und Fasern durch die Polymerisation von L-Alanin-NCA (ohne expliziten Initiatorzusatz) oder durch die Polymerisation von L-Leucin-NCA (Luftfeuchtigkeit als Initiator) mit in siedendem Benzol hergestellt (DuPont, 1957, US 2,789,973). Die Reaktionszeiten wurden mit 1-10 Tagen angegeben. Analog hierzu wurden auch von Ebert et al. die Herstellung von sehr hochmolekularen Polyaminosäuren beschrieben. Hierbei wurde durch Polymerisation in Benzol (70°C, ohne expliziten Initiatorzusatz) beispielsweise Poly-L-Leucin mit einem mittleren Molgewicht von 400000 g/mol hergestellt und bezüglich seiner Eigenschaften hinsichtlich der Herstellung von Fasern und Folien untersucht (Progr. Colloid &. Polymer Sci, 1976, 60, 183-193).

Auch für die Herstellung der benötigten N-Carboxyanhydride existieren eine Reihe von literaturbekannten Vorschriften (z.B. Rec. Trav. Chim. Pays-Bas, 1954, 73, 347).

Bei der Juliá-Colonna-Epoxidierung können jedoch nur bestimmte Polyaminosäuren als Katalysator verwendet werden, da die erreichbare Reaktionsgeschwindigkeit und der mögliche Enantiomerenüberschuß (ee) sehr stark von der verwendeten Polyaminosäure und der Art ihrer Herstellung abhängen (z.B. Bioorg. Med. Chem., 1999, 7, 2145-2156, Tetrahedron, 1984, 40, 5207-5211; Chirality, 1997, 9, 198-202). Im allgemeinen werden Polyaminosäuren mit einem mittleren Molgewicht von < 15000 g/mol verwendet. Die katalytische Aktivität der Polyaminosäure hängt auch in hohem Maße von der vorliegenden Konformation der Polyaminosäure ab, die wiederum von der Herstellungsmethode entscheidend beeinflusst wird (z.B. Bull. Chem. Soc. Jpn., 2000, 73, 2115-2121; J Org. Chem., 1993, 58, 6247-6254, Org. Lett., 2001, 3,683-686; *ibit* **2001,** *3,* 3839-3842). Meist werden heute bei der Juliä-Colonna-Epoxidierung D- oder L-Polyleucin als Katalysatoren verwendet. Andere Polyaminosäuren die mit Erfolg verwendet wurden sind beispielsweise D- bzw. L-Neopentylglycin (EP-A 1 006 127) oder D- oder L-Alanin.

Juliá fand heraus, dass durch Polymerisation von Alanin-NCA mit *n*-Butylamin eine Polyaminosäure erhalten werden kann, welche in der Lage ist, die enantioselektive Epoxidierung von Enonen zu katalysieren (Angew., Chem., Int. Ed. Eng., 1980, 19, 929-930). Die Reaktion wurde bei Raumtemperatur in Acetonitril geführt und erfolgte innerhalb von 4 Tagen. Nach einer Filtration des Polymers und einer Wäsche mit Ether konnte das Polymer in die Epoxidierung eingesetzt werden. Diese Vorschrift wurde von Juliá und Colonna mehrfach variiert (z.B. J. Chem. Soc., Perkin 1, 1982, 1317; Tetrahedron, 1983, 39, 1635; Tetrahedron, 1984, 40, 5207-5211), die bei Raumtemperatur ausgeführten Polymerisationen benötigten jedoch eine sehr lange Reaktionszeit. Ein weiterer deutlicher Nachteil des Verfahrens nach Juliá und Colonna war die schwierige Polymeraufarbeitung. Durch die Verwendung eines aminofunktionalisierten Polymers (Polystyrol) als Initiator konnten ähnlich aktive Polymere hergestellt werden, welche jedoch leichter handhabbar waren (J. Org. Chem., 1990, 55, 6047-6049). Die Polymerisation erfolgte nach dieser Vorschrift in Tetrahydrofuran bei Raumtemperatur über 40 h. Eine andere Vorschrift zur Herstellung von Polyaminosäuren (insbesondere Poly-L-Leucin, pLL) wurde von Ajinomoto Co., Inc., patentiert (JP 74 38,995) und von Flisak et al. in der Synthese von SK&F104353 genutzt *(*J. Org. Chem., 1993, 58, 6247-6254). Nach diesem Verfahren wurde N-Carboxyanhydrid im festen Zustand bei Raumtemperatur mit Luftfeuchtigkeit polymerisiert (5-10 Tage). Für eine gute katalytische Aktivität des Materials ist eine sehr hohe Reinheit des verwendeten N-Carboxyanhydrids zwingend notwendig. Die hohe Reinheit des hergestellten N-Carboxyanhydrids spielt auch bei der Methode nach Bentley et al. eine entscheidende Rolle *(*Chirality, 1997, 9, 198-202). Als Lösungsmittel für die Polymerisation wird auch hier THF beschrieben (Raumtemperatur, 3 Tage Reaktionszeit).

Neben der statistischen Polymerisation wurden auch Polyaminosäuren durch auf wendige, schrittweise Polymerisation in einem Peptidsynthesiser (unter Verwendung von Schutzgruppentechnik) bereitgestellt (z.B. Bull. Chem. Soc. Jpn., 2000, 73, 2115-2121; Tetrahedron Lett., 1998, 39, 9297-9300; WO-A-0194327).

Des weiteren wurden Polyaminosäuren für die Juliá-Colonna-Epoxidierung durch Polymerisation von N-Carboxyanhydriden mit aminosubstituierten Polyethylenglycolen hergestellt (z.B. WO-A-0194327). Auch hierbei werden lange Polymerisationszeiten benötigt (mehrere Tage).

Um die katalytische Aktivität von unterschiedlichen Polyaminosäurepräparationen zu vergleichen, wird zur Entwicklung und Beschreibung neuer Methoden in der Literatur ein Standardstestsystem mit einer Polyaminosäure als Katalysator und trans-Chalkon als Edukt verwendet. Aus der Reaktionsgeschwindigkeit und dem erhaltenen Enantiomerüberschuss (ee) kann die Qualität der Polyaminosäurepräparation abgeleitet werden.

Aufgabe der vorliegenden Erfindung ist es, Katalysatoren sowie Verfahren zur Herstellung der Katalysatoren bereitzustellen, die die vorstehend genannten Nachteile bei der enantioselektiven Epoxidierung nicht aufweisen. Von Bedeutung ist insbesondere eine einfache und reproduzierbare Herstellung der Katalysatoren. Von Bedeutung ist weiter eine hohe Aktivität, Stabilität, Raum-Zeit-Ausbeuten und Selektivität der Katalysatoren.

Überraschenderweise wurde nun gefunden, dass durch Umsetzung von Aminosäure-N-Carboxyanhydrid (Aminosäure-NCA) in aromatischen Lösungsmitteln, bei erhöhter Temperatur und in Gegenwart eines Initiators ein geeigneter Katalysator erhältlich ist. Gegenstand der Erfindung sind Polyaminosäuren u. Herstellverfahren gemäss den Ansprüchen 1 und 2, sowie die Verwendung von Polyaminosäuren in Epoxidierungsreaktionen gemäss Anspruch 3. Für die Ansprüche 1 und 2 sind die Initiatoren eingeschränkt wie in den Ansprüchen angegeben. Überraschenderweise wurde außerdem gefunden, dass die Isolierung des Katalysators wesentlich vereinfacht wird, wenn vor der Filtration C₁-C₄-Alkohol der Reaktionsmischung zugesetzt wird.

Im Folgenden wird die erfindungsgemäße Herstellung des Katalysators erläutert.

Beispielhaft kann die Herstellung des Katalysators durch folgendes Reaktionsschema dargestellt werden.

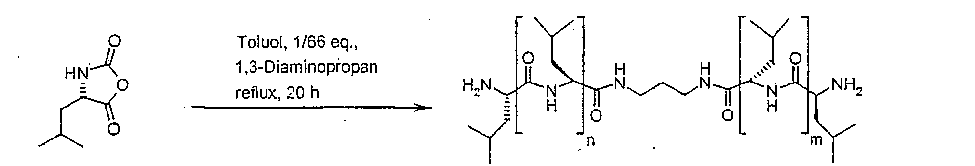

Die Herstellungszeiten des Katalysators können von Tagen auf wenige Stunden reduziert werden. Besonders überraschend wurde gefunden, dass der so hergestellte Katalysator eine wesentlich höhere katalytische Aktivität aufweist als Katalysatorpräparationen, die nach den bisher publizierten Verfahren hergestellt wurden. Darüber hinaus kann so der Katalysator in reproduzierbarer Qualität hergestellt werden.

Der Einfluss der Herstellungsweise auf die Aktivität des Katalysators wurde mit Hilfe der Standard-Testreaktion, die Polyaminosäure-katalysierte Epoxidierung von *trans-*Chalkon zu Epoxychalkon, belegt (dreiphasige Bedingung; vgl. Herstellungsbeispiele).

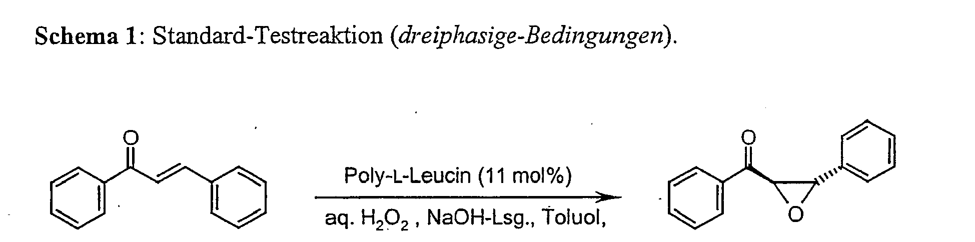

Als aromatische Lösungsmittel sind unsubstituierte, alkylierte, halogenierte und initiierte Benzolderivate geeignet. Hervorzuheben sind Benzol; Nitrobenzol, Alkylbenzole wie Toluol, o-, m-, p-Xylol, Kresol, Tetrahydronaphthalin; Halogenbenzole wie Chlor- und Dichlorbenzol. Besonders hervorzuheben sind Benzol, Toluol, Nitrobenzol und Chlorbenzol. Ganz besonders hervorzuheben ist Toluol. Gegebenenfalls können Lösungsmittelgemische verwendet werden.

Bevorzugt sind aromatische Lösungsmittel, in denen Edukt und Initiator unter den Reaktionsbedingungen löslich sind.

Die bekannten Aminosäure-NCA's können als Ausgangsmaterial für die Katalysatorherstellung verwendet werden. Insbesondere kommen die in der vorstehenden Literatur für die Juliá-Colonna-Epoxidierung beschriebenen Aminosäure-NCA's in Frage. Besonders bevorzugt sind D- bzw. L-Leucin-NCA, D- bzw. L-Alanin-NCA und D- bzw. L-Neopentylglycin-NCA. Ganz. besonders bevorzugt ist D- bzw. L-Leucin-NCA.

Die Herstellung der Aminosäure-NCA's ist bekannt bzw. kann analog zu bekannten Verfahren erfolgen.

Die Anfangskonzentration der Aminosäure-NCA's kann in einem freiten Bereich variiert werden. Im allgemeinen werden 0,5 bis 25 Massen-%, bevorzugt 1 bis 10 Massen-% und besonders bevorzugt 1 bis 5 Massen-% Aminosäure-NCA im Reaktionsgemisch verwendet.

Als Initiatoren können die bekannten Initiatoren verwendet werden. Insbesondere ein- und mehrwertige Alkohole, bzw. deren Salze, sowie ein- und mehrwertige Amine können verwendet werden. Besonders geeignet sind folgende Amine: 1,3-Diaminopropan, CLAMPS, n-Butylamin, amin-substituiertes PEG. Für die Ansprüche 1 und 2 gilt die dort angegebene Einschränkung hinsichtlich der Initiatoren.

Das molare Verhältnis von Aminosäure-NCA zu Äquivalent Initiator kann in einem breiten Bereich variiert werden und liegt zwischen 4:1 und 200:1. Bevorzugt liegt das Verhältnis zwischen 4:1 und 100:1; besonders bevorzugt zwischen 4:1 und 50:1, ganz besonders bevorzugt zwischen 10:1 und 40:1. Das Verhältnis variiert in Abhängigkeit vom verwendeten Initiator. Die durchschnittliche Kettenlänge kann beispielsweise durch den Initiator und das Verhältnis von Aminosäure-NCA zu Initiator beeinflusst werden.

Die Reaktionstemperatur kann in einem breiten Bereich variiert werden und liegt zwischen 30°C und dem Siedepunkt der Reaktionsmischung. Bevorzugt liegt die Reaktionstemperatur zwischen 50°C und der Siedetemperatur der Reaktionsmischung, besonders bevorzugt zwischen 80°C und 110°C, ganz besonders bevorzugt zwischen 90°C bis 110°C. Die Reaktionstemperatur kann im Reaktionsverlauf variiert werden. In einer Ausführungsform der Erfindung erfolgt eine Temperaturerhöhung nach dem Start der Reaktion. In einer alternativen Ausführungsform wird der Initiator in das siedende Lösungsmittel dosiert und die Reaktionsmischung während der gesamten Reaktionszeit auf Siedetemperatur gehalten.

Der Reaktionsdruck kann in einem breiten Bereich variiert werden und liegt zwischen 0,5 und 5 bar, bevorzugt zwischen 0,9 und 1,5 bar, besonders bevorzugt bei Normaldruck.

Die Isolierung des Katalysators aus dem Reaktionsgemisch kann nach laborüblichen Methoden erfolgen. So ist eine Abtrennung durch Filtration oder Zentrifugation möglich. Der so erhaltene Katalysator kann dann weitere Reinigungs- und Aufarbeitungsscliritte wie Waschen und Trocknen unterzogen werden. Vorteilhaft ist es, vor der Filtration oder Zentrifugation einen C₁-C₄-Alkohol zuzusetzen. Als C₁-C₄-Alkohol sind Methanol und Ethanol besonders geeignet. Die Mengen an zugesetzten Alkohol kann in einem breiten Bereich varriert werden und liegt zwischen 0,1:1 und 10:1 (v/v). Bevorzugte Bereiche liegen zwischen 0,5:1 und 2:1 (v/v) besonders bevorzugt ist das Volumenverhältnis 1:1.

Im Folgenden wird die erfindungsgemäße Verwendung des Katalysators in Epoxidierungsreaktionen erläutert.

Unter einer Epoxidierungsreaktion wird die Umsetzung einer C-C-Doppelbindung zu einem Oxiran verstanden. Insbesondere wird unter einer Epoxidierungsreaktion die Umsetzung von α,β-ungesättigten Enonen oder α,β-ungesättigten Sulfonen zu den entsprechenden Epoxiden verstanden.

Als α,β-ungesättigte Enone oder α,β-ungesättigte Sulfone können Verbindungen der allgemeinen Formel (II) eingesetzt werden 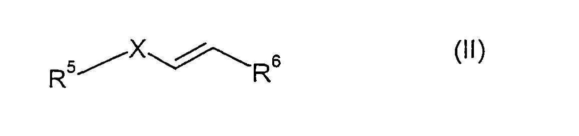 worin
- X: für (C=O) oder (SO₂) steht und
- R₅ und R₆: gleich oder verschieden sind und (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₇-C₁₉)-Aralkyl,(C₁-C₁₈)-Heteroaryl oder (C₂-C₁₉)-Heteroaralkyl bedeuten,
wobei die für R⁵ und R⁶ gennanten Reste einfach oder mehrfach mit gleichen oder verschiedenen Resten R⁷, Halogen, NO₂, NR⁷R⁸, PO₀₋₃R⁷R⁸, SO₀₋₃R⁷, OR⁷, CO₂R⁷, CONHR⁷ oder COR⁷ substituiert sein können und gegebenenfalls eine oder mehrere CH₂-Gruppen in den Resten R⁵ und R⁶ durch O, SO₀₋₂, NR⁷ oder PO₀₋₂R⁷ substituiert sind,
wobei R⁷ und R⁸ gleich oder verschieden sind und H, (C₁-C₁₈)-Alkyl, (C₂-C₁₈)-Alkenyl, (C₂-C₁₈)-Alkinyl, (C₃-C₈)-Cycloalkyl, (C₆-C₁₈)-Aryl, (C₁-C₁₈)-Heteroaryl, (C₁-C₈)-Alkyl-(C₆-C₈)-Aryl, (C₁-C₈)-Alkyl-(C₁-C₁₉)-Heteroaryl, (C₁-C₈)-Alkyl-(C₃-C₈)-Cycloalkyl bedeuten und diese Reste R⁷ und R⁸ einfach oder mehrfach mit gleichen oder verschiedenen Halogenresten substituiert sein können.

Unter einem (C₁-C₁₈)-Alkylrest wird im Rahmen der Erfindung ein Rest mit 1 bis 18 gesättigten C-Atomen verstanden, der beliebige Verzweigungen aufweisen kann. Insbesondere sind unter dieser Gruppe die Reste Methyl, Ethyl, *n*-Propyl, Isopropyl, *n*-Butyl, Isobutyl, *sec*-Butyl, *tert*-Butyl, Pentyl und Hexyl subsumierbar.

Ein (C₂-C₁₈)-Alkenylrest weist die für den (C₁-C₁₈)-Alkylrest genannten Merkmale auf, wobei innerhalb des Restes mindestens eine Doppelbindung vorhanden sein muss.

Ein (C₂-C₁₈)-Alkinylrest weist die für den (C₁-C₁₈)-Alkylrest genannten Merkmale auf, wobei innerhalb des Restes mindestens eine Dreifachbindung vorhanden sein muss.

Unter einem (C₃₋C₈)-Cycloalkylrest wird ein cylischer Alkylrest mit 3 bis 8 C-Atomen und gegebenenfalls beliebiger Verzweigung verstanden. Insbesondere zählen hierzu Reste wie Cyclopropyl, Cyclobutyl, Cyclopentyl, Cyclohexyl und Cycloheptyl. In diesem Rest kann eine oder mehrere Doppelbindungen vorhanden sein.

Unter einem (C₆-C₁₈)-Arylrest wird ein aromatischer Rest mit 6 bis 18 C-Atomen verstanden. Insbesondere zählen hierzu Reste wie Phenyl-, Naphthyl-, Anthryl- und Phenanthryl.

Unter einem (C₇-C₁₉)-Aralkylrest wird ein über einen (C₁-C₈)-Alkylrest an das Molekül gebundener (C₆-C ₁₈)-Arylrest verstanden.

Ein (C₁-C₁₈)-Heteroarylrest bezeichnet im Rahmen der Erfindung ein fünf-, sechs- oder siebengliedriges aromatisches Ringsystem mit 1 bis 18 C-Atomen, welches ein oder mehrere Heteroatome, bevorzugt N, O oder S im Ring aufweist. Zu diesen Heteroarylresten zählen z.B. 1-, 2-, 3-Furyl, 1-, 2-, 3-Pyrrol, 1-, 2-, 3-Thienyl, 2-, 3-, 4-Pyridyl, 2-, 3-, 4-, 5-, 6-, 7-Indolyl, 3-, 4-, 5-Pyrazolyl, 2-, 4-, 5-Imidazolyl, 1-, 3-, 4-, 5-Triazolyl, 1-, 4-, 5-Tetrazolyl, Acridinyl, Chinolinyl, Phenanthridinyl, 2-, 4-, 5-, 6-Pyrimidinyl und 4-, 5-, 6-, 7-(1-Aza)-indolizinyl.

Unter einem (C₂-C₁₉)-Heteroaralkylrest wird ein dem (C₇-C₁₉)-Aralkylrest entsprechendes heteroaromatisches System verstanden.

Unter Halogen oder auch Hal versteht man im Kontext dieser Erfindung Fluor, Chlor, Brom und Iod.

Die Menge der eingesetzten Polyaminosäure ist nicht kritisch und liegt üblicherweise im Bereich von 0.001-40 mol-%, bevorzugt im Bereich von 0,01-20 mol-%, besonders bevorzugt im Bereich von 0,01-10 mol-%, jeweils bezogen auf das eingesetzte α,β-ungesättigte Enon oder α,β-ungesättigte Sulfon.

### Beispiele

### 1. Herstellung des Ausgangsmaterials

L-Leucin-NCA: In einer Standard-Phosgenapparatur bestehend aus einem 2000 ml Vierhalskolben mit KPG-Rührer wurden 200.0 g (1.52 mol) L-Leucin in 2000 ml THF vorgelegt. Anschließend wurden 514.28 g (5.2 mol) Phosgen innerhalb von 6.5 h bei einer Temperatur von 22-33°C eingeleitet. Die klare Reaktionslösung wurde noch 16 h bei Raumtemperatur nachgerührt. Das Lösungsmittel wurde dann bei 35°C und 80 mbar komplett abdestilliert. Der Rückstand wurde portionsweise mit insgesamt 1800 ml n-Hexan gewaschen und bei Raumtemperatur unter vermindertem Druck getrocknet. Ausbeute: 203.2 g (85%)

### 2. Herstellung des Katalysators

### 2.1 erfindungsgemäße Herstellung - Variante A

(leu)₁₀-NH-(CH₂)₃-NH-(leu)₁₀ (statistische Mischung, durchschnittliche Kettenlänge angegeben): Unter Argon wurden 100.0 g (636.25 mmol) L-Leucin-NCA in einem 2 l Dreihalskolben mit aufgesetztem Rückflusskühler (verschlossen mit einem Siliconöl-gefüllten Blasenzähler) und mechanischem Rührwerk in 700 ml wasserfreiem Toluol vorgelegt und anschließend auf 110°C erhitzt. Zu der schnell gerührten Lösung wurden dann bei 110°C 2.358 g (31.81 mmol) 1.3-Diaminopropan in 20 ml wasserfreiem Toluol vorsichtig und langsam zugetropft. Nach Abklingen der initial sehr starken Gas-Entwicklung wurde die Reaktionsmischung für weitere 16 h bei 110°C nachgerührt. Die Reaktionsmischung wurde auf Raumtemperatur abgekühlt, mit 700 ml Methanol versetzt und unter Rückfluss ausgerührt. Der so erhaltene weiße Feststoff wurde bei Raumtemperatur abfiltriert und ein zweites Mal mit 1000 ml Methanol unter Rückfluss ausgerührt und abfiltriert. Das so isolierte Polymer wurde anschließend unter reduziertem Druck im Vakuumtrockenschrank (50°C, ca. 15 mbar) über Nacht getrocknet. Ausbeute: 67.0 g

### 2.2 erfindungsgemäße Herstellung - Variante B

(leu)₃₃-NH-(CH₂)₃-NH-(leu)₃₃ (statistische Mischung, durchschnittliche Kettenlänge angegeben): Unter Argon wurden 38.0g L-Leucin-NCA in einem 2 l Zweihalskolben mit aufgesetztem Rückflusskühler (verschlossen mit einem Siliconöl-gefüllten Blasenzähler) und mechanischem Rührwerk vorgelegt und in 970 ml wasserfreiem Toluol gelöst. Zu der schnell gerührten. Lösung wurden bei Raumtemperatur 0.272 g 1.3-Diaminopropan (frisch von CaH₂ abdestilliert) in 20 ml wasserfreiem Toluol zugegeben. Nach Abklingen der initial starken Gas-Entwicklung wurde die Reaktionsmischung langsam zum Rückfluss erwärmt und für 16 h bei dieser Temperatur gehalten. Die Reaktionsmischung wurde auf Raumtemperatur abgekühlt und anschließend abzentrifugiert. Das so isolierte Polymer wurde unter reduziertem Druck getrocknet (50°C - 60°C, ca. 15 mbar). Zum Abschluss wurde das Polymer in einer Reibschale pulverisiert und nochmals unter reduziertem Druck über P₂O₅ getrocknet. Ausbeute: 25.4 g

### 2.3 bekannte Herstellung

### (vgl. Chirality,1997, 9, 198-202, Aufarbeitung modifiziert)

Unter Argon wurden 38 g L-Leucin-NCA in einem 2 l Zweihalskolben mit aufgesetztem Rückflusskühler (verschlossen mit einem Siliconöl-gefüllten Blasenzähler) und magnetischem Rührer vorgelegt und in 970 ml wasserfreiem Tetrahydrouran (THF) gelöst. Zu der schnell gerührten Lösung wurden bei Raumtemperatur 0.272 g 1.3-Diaminopropan (frisch von CaH₂ abdestilliert) in 20 ml wasserfreiem THF zugegeben. Die Reaktionsmischung wurde für 5 Tage bei Raumtemperatur gerührt (ca. 400 - 500 U/min). Nach beendeter Reaktionszeit erfolgte die Aufarbeitung analog der unter a) beschriebenen Vorschrift. Ausbeute: 24.9 g.

### 3. Katalytische Reaktionen

### allgemeine Vorschriften zur Epoxidierung

Der Verlauf der Epoxidierungen wurde mit HPLC bzw. DC überwacht, Licht wurde während der Epoxidierungen ausgeschlossen. Analytische Proben wurden vor den HPLC-Messungen durch einen Membranfilter filtriert.

### Beispiel 1

(3-phasige-Bedingungen; Chirality, 1997, 9, 198-202, Aufarbeitung modifiziert)

100 mg pll (nicht voraktiviert) wurden in einer Mischung aus 0.8 ml Toluol, 0.2 ml NaOH (5M, 4.2 eq.) und 0.2 ml H₂O₂ (30%, aq.) suspendiert. Diese Mischung wurde für 6 h mit ca. 1250 U/min gerührt. Anschließend wurden 0.24 mmol *trans-*Chalkon und weitere 0.5 ml H₂O₂ (30%, aq., Gesamt = 28.5 eq.) zugesetzt. Nach beendeter Reaktion (bzw. einer gewählten Reaktionszeit) wurde die Reaktionsmischung mit 2 ml EtOAc verdünnt und anschließend zentrifugiert. Der Überstand wurde dann langsam in eine gerührte, eiskalte wässrige NaHSO₃ Lösung eingetragen (4 ml, 20 %). Nach der Phasentrennung wurde die organische Phase getrocknet (Na₂SO₄) und unter reduziertem Druck eingeengt.

| **Nr.** | **Katalysator** | **Reaktionszeit (h)** | **Umsatz** (%) | **ee (%)** |
|---|---|---|---|---|
| 1 | hergestellt nach 2.1 | 1,5 | 30 | 87 |
| 2 | hergestellt nach 2.2 | 1,5 | 59 | 91 |
| 3 | hergestellt nach 2.3 | 1,5 | 2 | nicht bestimmt |

## Patentansprüche

1. Polyaminosäuren, erhältlich durch ein Verfahren umfassend die Umsetzung von Aminosäure-N-Carboxyanhydriden in Gegenwart eines Initiators, **dadurch gekennzeichnet, dass**
a) ein aromatisches Lösungsmittel verwendet wird;
b) die Reaktionstemperatur zwischen 30°C und Siedetemperatur des Reaktionsgemisches liegt;
c) das molare Verhältnis von Aminosäure-N-Carboxyanhydrid zu Initiator zwischen 4:1 und 200:1 liegt und
d) als Initiator ein- oder mehrwertige Alkohole, deren Salze oder 1,3-Diaminoprogram, n-Butylamin oder CLAMPS eingesetzt werden

2. Verfahren zur Herstellung von Polyaminosäuren umfassend die Umsetzung von Aminosäure-N-Carboxyanhydriden in Gegenwart eines Initiators, **dadurch gekennzeichnet, dass**
a) ein aromatisches Lösungsmittel verwendet wird;
b) die Reaktionstemperatur zwischen 30°C und Siedetemperatur des Reaktionsgemisches liegt;
c) das molare Verhältnis von Aminosäure-N-Carboxyanhydrid zu Initiator zwischen 4:1 und 200:1 liegt und
d) als Initiator ein- oder mehrwertige Alkohole, deren Salze oder 1,3- Diaminopropan, n-Butylamin oder CLAMPS eingesetzt werden.

3. Verwendung von Polyaminosäuren, erhältlich durch ein Verfahren umfassend die Umsetzung von Aminosäure-N-Carboxyanhydriden in Gegenwart eines Initiators, **dadurch gekennzeichnet, dass**
a) ein aromatisches Lösungsmittel verwendet wird;
b) die Reaktionstemperatur zwischen 30°C und Siedetemperatur des Reaktionsgemisches liegt und
c) das molare Verhältnis von Aminosäure-N-Carboxyanhydriden zu Initiator zwischen 4:1 und 200:1 liegt,
als Katalysatoren in Epoxidierungsreaktionen.

## Claims

1. Polyamino acids obtainable by a method comprising the reaction of amino acid-N-carboxy anhydrides in the presence of an initiator, **characterized in that**
a) an aromatic solvent is used;
b) the reaction temperature is between 30°C and the boiling point of the reaction mixture;
c) the molar ratio of amino acid-N-carboxy anhydride to initiator is between 4:1 and 200:1 and
d) monohydric or polyhydric alcohols, salts thereof or 1,3-diaminopropane, n-butylamine or CLAMPS are used as initiator.

2. Method for producing polyamino acids comprising the reaction of amino acid-N-carboxy anhydrides in the presence of an initiator, **characterized in that**
a) an aromatic solvent is used;
b) the reaction temperature is between 30°C and the boiling point of the reaction mixture;
c) the molar ratio of amino acid-N-carboxy anhydride to initiator is between 4:1 and 200:1 and
d) monohydric or polyhydric alcohols, salts thereof or 1,3-diaminopropane, n-butylamine or CLAMPS are used as initiator.

3. Use of polyamino acids obtainable by a method comprising the reaction of amino acid-N-carboxy anhydrides in the presence of an initiator, **characterized in that**
a) an aromatic solvent is used;
b) the reaction temperature is between 30°C and the boiling point of the reaction mixture, and
c) the molar ratio of amino acid-N-carboxy anhydrides to initiator is between 4:1 and 200:1,
as catalysts in epoxidation reactions.

## Revendications

1. Polyaminoacides, pouvant être obtenus par un procédé comprenant la transformation de N-carboxyanhydrides d'aminoacides en présence d'un initiateur, **caractérisés en ce que**
a) on utilise un solvant aromatique ;
b) la température de réaction est comprise entre 30°C et la température d'ébullition du mélange réactionnel ;
c) le rapport molaire de N-carboxyanhydride d'aminoacide à initiateur se situe entre 4:1 et 200:1 et
d) on utilise comme initiateur des alcools monovalents ou polyvalents, leurs sels ou le 1,3-diaminopropane, la n-butylamine ou un CLAMPS.

2. Procédé pour la préparation de polyaminoacides, comprenant la transformation de N-carboxyanhydrides d'aminoacides en présence d'un initiateur, **caractérisé en ce que**
a) on utilise un solvant aromatique ;
b) la température de réaction est comprise entre 30°C et la température d'ébullition du mélange réactionnel ;
c) le rapport molaire de N-carboxyanhydride d'aminoacide à initiateur se situe entre 4:1 et 200:1 et
d) on utilise comme initiateur des alcools monovalents ou polyvalents, leurs sels ou le 1,3-diaminopropane, la n-butylamine ou un CLAMPS.

3. Utilisation de polyaminoacides, pouvant être obtenus par un procédé comprenant la transformation de N-carboxyanhydrides d'aminoacides en présence d'un initiateur, **caractérisée en ce que**
a) on utilise un solvant aromatique;
b) la température de réaction est comprise entre 30°C et la température d'ébullition du mélange réactionnel et
c) le rapport molaire de N-carboxyanhydrides d'aminoacide à initiateur se situe entre 4:1 et 200:1,
comme catalyseurs dans des réactions d'époxydation.
